# EUROPEAN PATENT APPLICATION

(11) **EP 2 407 444 A2**
(43) Date of publication of application: **18.01.2012**
(21) Application number: 11173087.5
(22) Date of filing: 18.03.2009
(51) Int. Cl.: C07C 29/16, C07C 31/133, C07C 45/53, C07C 49/413, C07C 51/31, C07C 53/126

(54) **Process for the preparation of dodecanedioic acid**

(30) Priority: 19.03.2008 US 37861 P
(62) Divisional of application: 09721453.0
(71) Applicant: Invista Technologies S.a r.l., 9000 St. Gallen (CH)
(72) Inventor: Rajendran, Gurusamy, League City, TX 77573 (US)
(74) Representative: Cockerton, Bruce Roger

(57) **Abstract**

The present disclosure provides processes for the preparation of dodecanedioic acid (DDDA)

## Description

### CROSS-REFERENCE TO RELATED APPLICATION

This application claims priority to U.S. provisional application entitled, "CYCLODODECATRIENE AND CHEMICAL PROCESS", having serial number 61/037,861, filed on March 19, 2008, which is entirely incorporated herein by reference.

### FIELD OF THE INVENTION

The disclosure herein relates to a process for forming a mixture containing dodecanedioic acid (DDDA).

### BACKGROUND

It is known to produce dodecanedioic acid (DDDA) using a chemical process starting with the conversion of 1,3-butadiene (trimerization) to 1,5,9-cyclododecatriene (CDDT). This trimerization process employs a Ziegler-Natta catalyst (TiCl₄ and aluminum chloride) and mild 70 to 80°C conditions at ca. 2 bar pressure absolute. Followed by reduction of the CDDT to cyclododecane using hydrogen and Raney Nickel catalyst at 170 to 180°C and 26 - 28 bar pressure absolute, the cyclododecane is oxidized with air to a mixture comprising cyclododecanol (CDDA) and cyclododecanone (CDDK) in the presence of a boric acid catalyst at 160 to 180°C and 1 - 2 bar pressure absolute. The CDDK and CDDA mixture contains about 80-90% CDDA and 10-20% CDDK. This mixture is oxidized using nitric acid and catalysts comprising copper and vanadium to obtain a mixture comprising DDDA.

### SUMMARY

Briefly described, embodiments of this disclosure include processes of making dodecanedioic acid (DDDA), and the like. One exemplary process for making dodecanedioic acid, among others, includes: contacting 1,3-butadiene with a first catalyst and forming cyclododeca-1,5,9-triene; oxidizing the cyclododeca-1,5,9-triene using an oxygen containing reagent to form epoxycyclododeca-5,9-diene, converting the epoxycyclododeca-5,9-diene into a first mixture comprising cyclododecanol and cyclododecanone; and contacting the first mixture with reactants comprising a third catalyst and nitric acid to form a second mixture comprising dodecanedioic acid.

One exemplary process for making laurolactone, among others, includes: providing a reaction vessel including a solution of cyclododecanone (CDDK) that is dissolved in about an equal weight of an acid anhydride; providing to the vessel an acid catalyst in an amount effective to promote the reaction, wherein the acid catalyst is selected on the basis of its pKₐ such that its pKₐ is about 0 to about 5; and introducing hydrogen peroxide to the reaction vessel to promote the Baeyer-Villiger oxidation, wherein about 20% or more of the cyclododecanone (CDDK) is converted to laurolactone.

One exemplary process for making laurolactone, among others, includes: contacting 1,3-butadiene with a first catalyst and forming cyclododeca-1,5,9-triene; oxidizing the cyclododeca-1,5,9-triene using an oxygen containing reagent to form epoxycyclododeca-5,9-diene, converting the epoxycyclododeca-5,9-diene into a first mixture comprising cyclododecanol and cyclododecanone; disposing the cyclododecanone (CDDK) from the first mixture in a reaction vessel, wherein the CDDK is dissolved in about an equal weight of an acid anhydride; providing to the vessel an acid catalyst in an amount effective to promote the reaction, wherein the acid catalyst is selected on the basis of its pKₐ such that its pKₐ is about 0 to about 5; and introducing hydrogen peroxide to the reaction vessel to promote the Baeyer-Villiger oxidation, wherein about 20% or more of the (CDDK) is converted to laurolactone.

### DETAILED DESCRIPTION

Before the present disclosure is described in greater detail, it is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

Embodiments of the present disclosure will employ, unless otherwise indicated, techniques of chemistry, chemical engineering, and the like, which are within the skill of the art. Such techniques are explained fully in the literature.

It must be noted that, as used in the specification and the appended claims, the singular forms "a", "an", and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

### Discussion

Embodiments of the present disclosure provide for a process for making dodecanedioic acid (DDDA). Embodiments of the present disclosure may have certain advantages in energy consumption versus other methods. In general, embodiments of the present disclosure require less energy in the conversion of cyclododeca-1,5,9-triene to cyclododecanol (CDDA) or cyclododecanone (CDDK) in comparison to a process disclosed by Welton in his U.S. Patent Number 3,607,948 (assigned to E. I. du Pont de Nemours and Co.); the disclosure of Welton are herein incorporated by reference in their entirety.

In general, embodiments of this disclosure relate to processes for the preparation of dodecanedioic acid (DDDA) by oxidizing cyclododeca-1,5,9-triene with an oxygen containing reagent to form epoxycyclododeca-5,9-diene, forming a mixture containing cyclododecanol and cyclododecanone, and oxidizing the mixture with nitric acid to form a DDDA containing mixture of diacids.

Embodiments of this disclosure relate to processes of chemical transformation employing the steps of polymerizing (trimerization) of 1,3-butadiene to cyclododeca-1,5,9-triene, which is subsequently oxidized to form epoxycyclododeca-5,9-diene. Then, epoxycyclododeca-5,9-diene is converted by selective reduction and/or rearrangement to a first mixture containing cyclododecanol (CDDA) and cyclododecanone (CDDK). Next, the first mixture is oxidized to a second mixture comprising dodecanedioic acid (DDDA). Reduction of the cyclododeca-1,5,9-triene (CDDT) to cyclododecane with hydrogen over a heterogeneous Raney Nickel catalyst is not performed in this process and no catalyst is employed in the intermediate steps of this transformation as compared with the known method of employing air oxidation in the presence of boric acid. Ways to perform the oxidation of cyclododeca-1,5,9-triene to the epoxycyclododeca-5,9-diene are known from T. Sridhar *et al. (Department of Chemical Engineering, Monash University, Victoria 3800, Australia)* found in Ind. Eng. Chem. Res., 46 (10), 3057 -3062, 2007 "Uncatalyzed Oxidation of 1,5,9-Cyclododecatriene with Molecular Oxygen."

In an embodiment, the process includes contacting 3-butadiene (BD) with a first Ziegler-Natta type catalyst effective for trimerizing BD and forming cyclododeca-1,5,9-triene (CDDT). Next, the process includes oxidizing the cyclododeca-1,5,9-triene (CDDT), using an oxygen containing reagent, to form epoxycyclododeca-5,9-diene (ECDDD). In an embodiment, the oxidization can be conducted in the presence of a second catalyst. Subsequently, the process includes converting epoxycyclododeca-5,9-diene (ECDDD) into a first mixture comprising cyclododecanol (CDDA) (*e.g.*, about 30% to 70% of the first mixture) and cyclododecanone (CDDK) (*e.g.*, about 70% to 30% of the first mixture). The conversion can be conducted using selective reduction and/or rearrangement. Next, the process includes contacting the first mixture with a third catalyst (*e.g.*, ammonium meta-vanadate and copper nitrate) and nitric acid, which results in forming a second mixture comprising dodecanedioic acid (DDDA) (*e.g.*, about 40% to 90% of the second mixture).

In an embodiment, a process for making dodecanedioic acid can include contacting 1,3-butadiene with a first catalyst effective for trimerization and forming cyciododeca-1,5,9-triene (CDDT) and minor amounts of 1,3-butadiene dimers, *i.e.*, cylcooctadiene and vinyl cyclohexene.

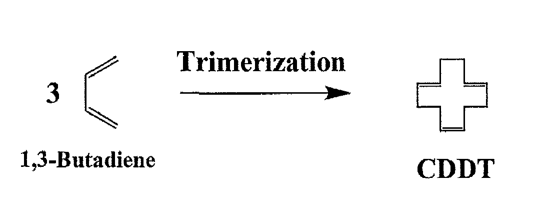

In an embodiment, the first catalyst (also referred to as a "trimerization catalyst") may be a homogeneous Ziegler-Natta type; *e.g.*, TiCl₄ and aqueous aluminum chloride (AICI), or diethyl-AICI.

Next, the process includes oxidizing the cyclododeca-1,5,9-triene using an oxygen containing reagent to form epoxycyclododeca-5,9-diene. In an embodiment, a second catalyst (*e.g.*, hydrocarbon soluble Mo, V, or Ti naphthenates) can be used to form epoxycyclododeca-5,9-diene. However, in an embodiment, a second catalyst is not required to form epoxycyclododeca-5,9-diene.

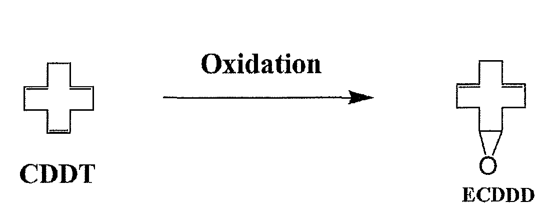

Subsequently, the process includes converting the epoxycyclododeca-5,9-diene into a first mixture comprising cyclododecanol and cyclododecanone. In an embodiment, the conversion can be performed by selective reduction and rearrangement such as those listed here:

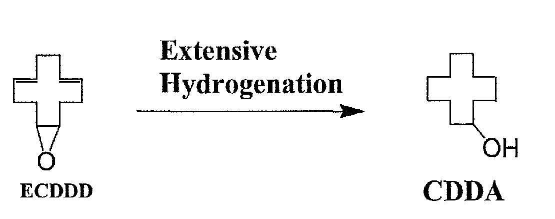

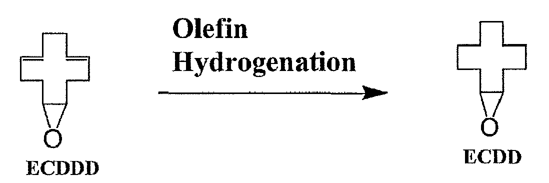

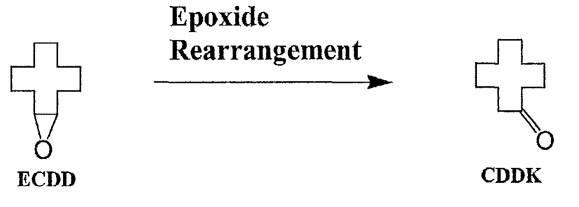

Next, the process includes contacting the first mixture with a third catalyst (ammonium meta-vanadate and copper nitrate) and nitric acid (*e.g.*, concentrated nitric acid), which forms dodecanedioic acid (and minor amounts of linear dicarboxylic acids including those of fewer than 12 carbon atoms).

This mixture (second mixture) resulting from the nitric oxidation contains the C₁₁ dicarboxylic acid (*e.g.*, about 1% to 5% of the second mixture), as well as, smaller amounts of the C₆ to C₁₀ dicarboxylic acids (*e.g.*, about 1% to 5% of the second mixture).

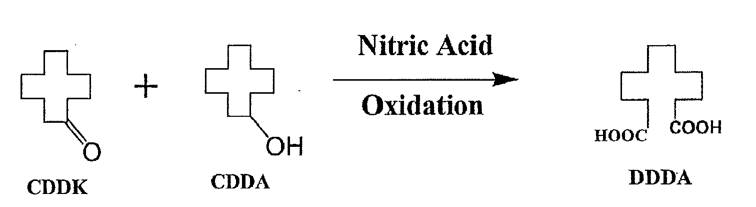

In an embodiment, the oxidation process of cyclododeca-1,5,9-triene to epoxycyclododeca-5,9-diene and other mono-oxygen substituted cyclododecatrienes (*e.g.*, alcohol and ketone substituents), herein disclosed, may be achieved by semi-continuous and continuous processes in the presence of oxygen gas. In an embodiment, the oxygen gas is about 1 and 99.5% of the gas, with the remaining balance being nitrogen gas. In an embodiment, the product yields can be about 60 and 96%, while conversion to product is about 10 and 99%.

In an embodiment, the process for oxidation of cyclododeca-1,5,9-triene to epoxycyclododeca-5,9-diene and other mono-oxygen substituted cyclododecatrienes, herein disclosed, is achievable in semi-continuous and continuous processes in the presence of oxygen gas. In an embodiment, the oxygen gas is about 1 and 99.5%, and the remaining balance being a synergistic gas such as carbon dioxide or an inert gas such as argon. It should be noted that synergistic gases can enhance the solubility of oxygen at temperatures and pressures where nitrogen provides no such synergy in enhancing solubility of the oxygen. In an embodiment, the enhanced solubility of oxygen in cyclododeca-1,5,9-triene results in higher conversion and selectivity to product. In an embodiment, the product yields achievable are about 80 and 96%, while the conversion to product is about 40 and 99%.

In an embodiment, the process provided herein for preparing epoxycyclododeca-5,9-diene and other mono-oxygen substituted cyclododecatrienes is achievable by reacting cyclododeca-1,5,9-triene with 99.9% pure oxygen at temperatures of about 50°C to 150°C and at pressures of about 50 psig to 250 psig.

In an embodiment, the process provided herein for preparing epoxycyclododeca-5,9-diene along with other mono-oxygen substituted cyclododecatrienes by reacting cyclododeca-1,5,9-triene with air at temperatures of about 60°C to 220°C and at pressures of about 60 psig to 500 psig.

In an embodiment, the process provided herein for preparing epoxycyclododeca-5,9-diene along with other mono-oxygen substituted cyclododecatrienes is achievable by reacting cyclododeca-1,5,9-triene with a mixture of air and pure oxygen or nitrogen. In an embodiment, the mixture includes air enriched with pure oxygen or pure oxygen and pure nitrogen providing the balance of the composition. In an embodiment, the combination of air and pure oxygen comprise a composition providing oxygen to the reaction in an amount from about 1% to about 99%. Similarly, the combination of pure nitrogen and pure oxygen may comprise a composition providing oxygen to the reaction in an amount from about 1% to about 99%. In an embodiment, the reaction temperature for this process can be about 50°C to 220°C at pressures of about 50 psig to 500 psig.

In an embodiment, the process provided herein for preparing epoxycyclododeca-5,9-diene along with other mono-oxygen substituted cyclododecatrienes is achievable, in a continuous process, by reacting cyclododeca-1,5,9-triene with a mixture of air and pure oxygen or pure oxygen and nitrogen. In an embodiment, the mixture includes air enriched with pure oxygen or pure oxygen and pure nitrogen providing the balance of the composition. In an embodiment, the combination of air and pure oxygen comprise a composition providing oxygen to the reaction in an amount from about 1% to about 99%. Similarly, the combination of pure nitrogen and pure oxygen may comprise a composition providing oxygen to the reaction in an amount from about 1% to about 99%. In an embodiment, this step can be conducted in the presence of initiators chosen from among AIBN, organic peroxides (*e.g.*, t-butyl hydroperoxide), or other azo-initiators (*e.g.*, t-butyl carbonyl azide). However, the presence of the initiators is not required.

In an embodiment, the process provided herein for preparing epoxycyclododeca-5,9-diene along with other mono-oxygen substituted cyclododecatrienes is achievable by reacting cyclododeca-1,5,9-triene with a mixture of air and pure oxygen or pure oxygen and nitrogen. In an embodiment, the mixture includes air enriched with pure oxygen or pure oxygen and pure nitrogen providing the balance of the composition. In an embodiment, the combination of air and pure oxygen comprise a composition providing oxygen to the reaction in an amount from about 1% to about 99%. Similarly, the combination of pure nitrogen and pure oxygen may comprise a composition providing oxygen to the reaction in an amount from about 1 % to about 99%. In an embodiment, this step can be conducted with the aid of a metal catalyst such as silver, nickel, iron and/or cobalt. In an embodiment, this step is not conducted with a metal catalyst.

In an embodiment, the process provided herein for preparing epoxycyclododeca-5,9-diene and other mono-oxygen substituted cyclododecatrienes is achievable by reacting cyclododeca-1,5,9-triene (CDDT) with a mixture of air and pure oxygen or pure oxygen and nitrogen. In an embodiment, the mixture includes air enriched with pure oxygen or pure oxygen and pure nitrogen providing the balance of the composition. In an embodiment, the combination of air and pure oxygen comprise a composition providing oxygen to the reaction in an amount from about 1% to about 99%. Similarly, the combination of pure nitrogen and pure oxygen may comprise a composition providing oxygen to the reaction in an amount from about 1% to about 99%. In an embodiment, the reaction can be carried out continuously using a fixed bed catalyst chosen from among of the metals: silver, nickel, iron and/or cobalt (the active metal supported on a catalyst support can include aluminum oxide, silicon dioxide or activated carbon).

In an embodiment, this continuous process can be advantageously carried out at with varying CDDT feed rates. Useful feed rates for CDDT are about 1 liter per hour to 5 liters per hour in a continuously circulating process stream. In an embodiment, the residence time is advantageously varied from about 2 minutes to 150 minutes and for times of about 10 minutes to 120 minutes. In an embodiment, the reaction temperature can advantageously be about 50°C to 200°C, and the temperature can be about 80°C to 160°C. The yields of epoxycyclododeca-5,9-diene and other mono-oxygen substituted cyclododecatrienes can be about 60 to 99% with a conversion of the starting material cyclododeca-1,5,9-triene to products of about 10 to 99%.

The process provided herein for preparing cyclododecanol (CDDA) is achievable using noble metal catalysts (Pd, or Pt supported on appropriate supports including, but not limited to, aluminum oxide, silicon dioxide and activated carbon) at hydrogen gas pressures of about 500 psig to 4500 psig. Starting with epoxycyclododeca-5,9-diene along with mono-oxygen substituted cyclododecatrienes a substantially complete hydrogenation to cyclododecanol (CDDA) is achievable. In an embodiment, the CDDA yields in the process can be about 96% to 99.9% with a conversion to product about 95% to 99%.

In an embodiment of the present disclosure, a mixture of cyclododecanone (CDDK), which may be obtained from an embodiment of the present disclosure described above, can be used to make laurolactone. In an embodiment, the process includes providing to a reaction vessel including a solution of cyclododecanone (CDDK) that is dissolved in a substantially equal weight of an acid anhydride (*e.g.*, acetic anhydride, maleic anhydride, or the like) and providing to the vessel an acid catalyst. In an embodiment, the acid catalyst can be provided in an amount effective to promote the reaction, *e.g.*, 0.5% to 10% by weight based on the amount of CDDK present. In an embodiment, the acid catalyst can be selected on the basis of its pKₐ such that its pKₐ is about 0 to about 5 (*e.g.*, dichloroacetic acid or a peracid such as trifluoroperacetic acid, and the like). In addition, the process includes introducing hydrogen peroxide (*e.g.*, 1 to 10 moles hydrogen peroxide per mole of CDDK present or a similar amount of peracid such as trifluoroperacetic acid, for example) to the vessel and promoting the Baeyer-Villiger oxidation of cyclododecanone (CDDK) and converting a substantial portion (*e.g.*, about 20% or more of the CDDK is converted) of the cyclododecanone (CDDK) to laurolactone.

### TEST AND ANALYTICAL METHODS

In general, oxidation of cyclododeca-1,5,9-triene in foregoing process scheme may be monitored by the rate of uptake of oxygen, as well as, by taking samples intermittently during the reaction. It is convenient, as one skilled in the art would know, to analyze the samples taken intermittently or in the case of final reaction products by chromatographic methods, *e.g.*, GC, GC-MS, HPLC and by spectroscopic methods, *e.g.*, ¹H NMR and ¹³C NMR, and also infrared (IR) methods.

All gas chromatographic (GC) analysis may be performed using an AGILENT TECHNOLOGIES 6890 equipped with AGILENT DB-FFAP column (25m x 0.20 mm x 0.3 µm) with injector and detector temperatures kept at 250°C. Such samples are conveniently analyzed using hexadecane as an internal standard at 120°C isothermal with helium as a carrier gas.

Gas chromatography mass spectrometry (GC-MS) analysis is performed on an AGILENT TECHNOLOGIES 6890 with a Model 5973-MSD (mass-selective detector) equipped with an Agilent DB-FFAP column (25m x 0.20 mm x 0.3 µm). The injector and detector temperatures are maintained at 250°C. The samples are analyzed using hexadecane as the internal standard at 120°C isothermal with helium as the carrier gas.

Proton (¹H) NMR and Carbon (¹³C) NMR is done using a Varian 500 MHz NMR equipped with tuning probe. Selective decoupling and homo- and hetero-nuclear NOE experiments may be performed to establish the geometry and positions of oxygen groups in the product.

Pressures reported herein refer to pounds per square inch gauge (psig) which include the pressure of one atmosphere (14.7 pounds per square inch).

Absolute pressures in kilopascals (kPa) are referenced from vacuum. One pound per square inch is about 6.9 kPa.

### EXAMPLES

Now having described the embodiments of the present disclosure, in general, the following Examples describe some additional embodiments of the present disclosure. While embodiments of the present disclosure are described in connection with the following examples and the corresponding text and figures, there is no intent to limit embodiments of the present disclosure to this description. On the contrary, the intent is to cover all alternatives, modifications, and equivalents included within the spirit and scope of embodiments of the present disclosure.

### EXAMPLE 1.

In an example, cyclododeca-1,5,9-triene (40.5 grams, 0.25 mole) is introduced to an autoclave reactor along with a suitable initiator (0.35% w/w). The autoclave is sealed and the internal air is displaced by introducing nitrogen gas. The autoclave is provided with agitation and heating to 100°C. After the autoclave reactor temperature is attained (100°C) the nitrogen gas is displaced with pure oxygen gas at a pressure of 80 pounds per square inch gage (psig), ca. 450 kPa absolute. The reaction is run in a semi-continuous mode. The pressure in the reactor is maintained continuously with the aid of a control valve set at 80 psig while the outlet valve from the reactor is closed. The reaction temperature is controlled at 100 +/- 3°C with the aid of a cooling loop inside the reactor. Samples are taken intermittently to monitor the progress of the reaction. Each sample is quantitatively analyzed by the analytical methods disclosed above. The reaction is stopped at the end of 2.5 hours, cooled to below 30°C and vented to ambient pressure. The contents are transferred to glass vessel for peroxide decomposition and product isolation. The selectivity of this reaction to epoxy-cyclododeca-6-10-diene and other mono-oxygenated products like alcohol (CDDA) and ketone (CDDK) is 96%. The conversion of cyclododeca-1,5,9-triene to the epoxide (ECDDD) and mono-oxygenated products is 88%.

### EXAMPLE 2.

In another example; cyclododeca-1,5,9-triene (40.5 grams, 0.25 mole) is charged into an autoclave reactor along with an initiator (0.35% w/w); as in Example 1. The autoclave is sealed and the air displaced by nitrogen. Agitation is started and the autoclave heated to 110°C. After the reactor temperature attain, the 110°C nitrogen gas is flushed with pure oxygen and pressurized to 100 psig; ca. 790 kPa. The reaction is run in a semi-continuous mode. The pressure in the reactor is maintained continuously with the aid of a control valve set at 110 psig, while an outlet valve from the reactor is closed. The reaction temperature may be controlled at 110 +/- 3°C with the aid of a cooling loop inside the reactor. Samples are taken intermittently and quantitatively analyzed by the foregoing analytical methods to monitor the progress of the reaction. The reaction is stopped at the end of 2 hours, cooled to < 30°C and vented to ambient pressure. The contents may be transferred to glass vessel for the peroxide decomposition and product isolation. The selectivity of this reaction to epoxy cyclododeca-6-10-diene and other mono-oxygenated products like alcohol and ketone is 91%. The conversion of cyclododeca-1,5,9-triene to the epoxide (ECDDD) and mono-oxygenated (CDDA, CDDK) products is 92%.

### EXAMPLE 3.

In another example, Example 2 is followed identically with the exception that the reaction is stopped at the end of 3.5 hours, cooled to < 30°C and vented to ambient pressure. In this example, the selectivity of the oxidation reaction to epoxy cyclododeca-6-10-diene and other mono-oxygenated products alcohol (CDDA) and ketone (CDDK) formation is 95%. The conversion of CDDT to epoxide (ECDDD) and mono-oxygenated products (CDDA, CDDK) is 81 %.

### EXAMPLE 4.

In another example, Example 2 is followed identically with the following exceptions. The oxidation of cyclododeca-1,5,9-triene is conducted at temperature controlled to 115 +/- 3°C and the reaction is stopped at the end of 3 hours, cooled to < 30°C and vented to ambient pressure. The contents were transferred to glass vessel for the peroxide decomposition and product isolation. The selectivity of this reaction to epoxy cyclododeca-6-10-diene and other mono-oxygenated products alcohol (CDDA) and ketone (CDDK) is 93%. The conversion of CDDT to epoxide (ECDDD) and mono-oxygenated products (CDDA, CDDK) is 84%.

### EXAMPLE 5.

In another example, cyclododeca-1,5,9-triene (40.5 grams, 0.25 mole) is charged to an autoclave reactor along with an initiator (0.35% w/w). The autoclave is sealed and the air is displaced by nitrogen gas. Agitation is started and the autoclave is heated to 100°C. After the reactor temperature attains 100°C, the nitrogen gas is flushed out with nitrogen and oxygen gas mixture (1:1 by weight %) and pressurized to 100 psig, ca. 790 kPa. The reaction is run in a semi-continuous mode. The pressure in the reactor is maintained continuously with the aid of a control valve set at 100 psig and a reactor outlet vent valve is set to relieve an amount of gas equivalent to the amount of gas added to the reactor. The reaction temperature is controlled at 100 +/- 3°C with the aid of a cooling loop inside the reactor. Samples taken intermittently are quantitatively analyzed by the analytical methods above to monitor the progress of the reaction. The reaction is stopped at the end of 3 hours, cooled to < 30°C and vented to ambient pressure. The contents are transferred to a glass vessel for the peroxide decomposition and product isolation. The selectivity of this reaction to epoxy cyclododeca-6-1 0-diene and other mono-oxygenated products alcohol (CDDA) and ketone (CDDK) is 96%. The conversion of CDDT to epoxide (ECDDD) and mono-oxygenated products (CDDA, CDDK) is 86%.

### EXAMPLE 6.

In an example of an oxidation process, feeding cyclododeca-1,5,9-triene, heated to 100°C, by means of a pump to a top portion of a trickle bed reactor column with inert packing is performed continuously. Oxygen gas preheated at 100 °C is pumped into the trickle bed reactor at two different levels in the lower half of the column. The pump at the bottom of the trickle bed reactor circulates liquid from heat exchanger means. A slip stream sample from the circulation loop, equivalent to the amount of fresh feed of cyclododeca-1,5,9-triene is continuously taken out as a reaction product. The pressure in the reactor is maintained at 100 psig (ca. 790 kPa absolute) throughout the continuous oxidation reaction by means of a control valve at a column vent line. The conversion of cyclododeca-1,5,9-triene into products (ECDDD, CDDA, CDDK) is 94% and selectivity is 96% at a best selected feed rate.

### EXAMPLE 7.

In yet another example of a continuous oxidation process, cyclododeca-1,5,9-triene (CDDT) and oxygen gas are preheated separately to 100°C and pumped into the bottom of a bubble column reactor. The pump at the bottom of the bubble column provides circulation to the liquid after passing through a heat exchanger. A slip stream sample from the pump circulation loop, equivalent to the amount of cyclododeca-1,5,9-triene introduced is continuously taken out as reaction product. The pressure in the reactor is maintained at 100 psig (ca. 790 kPa absolute) throughout the continuous oxidation reaction by means of a control valve in a column vent line. The cyclododeca-1,5,9-triene rate of feed is varied in order to provide different residence times for the reaction. The conversion of cyclododeca-1,5,9-triene into products (ECDDD, CDDA, CDDK) is 96% and selectivity is 97% under a best selected feed rate for the CDDT.

### EXAMPLE 8.

In yet another example of a continuous oxidation process, cyclododeca-1,5,9-triene (CDDT) and air are preheated separately to 110°C and pumped to the top of a trickle bed column reactor with inert packing. The air preheated to 110°C is pumped to the trickle bed reactor at two different levels at the lower third of the column. The pump at the bottom of the reactor circulates the liquid passing through a heat exchanger means. A slip stream sample from the circulation loop equivalent to the amount of fresh feed of cyclododeca-1,5,9-triene is withdrawn continuously as reaction product. The pressure in the reactor is maintained at 120 psig (ca. 930 kPa absolute) for this continuous reaction by means of a control valve in a vent line of the reactor. The conversion of cyclododeca-1,5,9-triene into products (ECDDD, CDDA, CDDK) is 91 % and selectivity is 94% under best selected feed rate for the CDDT.

### EXAMPLE 9.

In yet another example of a continuous oxidation process, CDDT (cyclododeca-1,5,9-triene) and air preheated separately to 110°C are pumped into the bottom of a bubble column reactor. The pump at the bottom of the bubble column circulates the liquid passing through heat exchanger means. A slip stream sample taken from the circulation loop equivalent to the amount of fresh feed of CDDT is continuously withdrawn as reaction product. The pressure in the reactor is maintained at 110 psig (ca. 860 kPa absolute) throughout this continuous reaction by means of a control valve in the reactor vent line. The CDDT feed rate is varied in order to provide different residence times for the reaction. The conversion of cyclododeca-1,5,9-triene into products (ECDDD, CDDA, CDDK) is 93% and selectivity is 96% under a selected best feed rate for CDDT.

### EXAMPLE 10.

The reaction products from Examples 1 to 8 are collected and individually distilled in a glass apparatus under reduced pressure. The small fraction collected at 130°C comprises predominately the unreacted cyclododeca-1,5,9-triene. The main fraction collected from 145 - 155°C will comprise predominately epoxycyclododeca-5,9-diene and minor amounts of cyclododeca-2,6,10-trienol and cyclododeca-2,6,10-trienone.

### EXAMPLE 11.

In an example, 40 grams of product collected from a process according to Example 8 are subjected to a distillation process according to Example 10. A fraction collected between 145 - 155°C is transferred to a high pressure autoclave. A hydrogen reduction catalyst comprising palladium on carbon is introduced. The autoclave is sealed, the air is displaced completely with hydrogen gas and the autoclave is heated to 220°C. Additional hydrogen gas is provided and the pressure is maintained at 1300 psig (ca. 9000 kPa absolute) for 12 hours. The autoclave is cooled to 100°C, a sample is taken for analysis and transferred into a glass reactor prior to solidification. The sample taken is identified as cyclododecanol (CDDA) having a purity of 98.5%, the yield is 97%.

### EXAMPLE 12.

In an example, cyclododecanol (CDDA) is dehydrogenated continuously with the aid of a fixed bed catalyst. A catalyst comprising Raney-Nickel or a substantial equivalent catalyst will effect dehydrogenation of CDDA to cyclododecanone (CDDK). The dehydrogenation temperature range is selected to be between 120°C and 350°C. The yield of CDDK is 98% at the best selecting dehydrogenation conditions.

### EXAMPLE 13.

In an example, cyclododecanone (CDDK) (36.8 g, 0.20 moles) is heated with an excess of nitric acid and a V/Cu catalyst (*e.g.*, ammonium vanadate and copper nitrate) in a glass reactor for 1 hour. A portion of the reaction work-up is evaporated and then allowed to cool. A product of the reaction is identified as dodecanedioic acid (DDDA) after crystallization from solution. The crystallized DDDA is further filtered and washed with cold water. A purity for the DDDA is expected to be 99% and the yield based on cyclododecanol is 88%. The filtrate upon further evaporation and crystallization will provide another crop of crystals comprising mixed dicarboxylic acids of 12, 11, 10, 9, 8 and 7 carbon atoms. The combined overall yields of DDDA and mixed di-acids based on CDDK are about 94%.

### EXAMPLE 14.

In an example, cyclododecanone (CDDK) (36.8 g, 0.20 moles) is dissolved in an equal weight of acetic anhydride (in an embodiment, maleic anhydride may be an equally effective acid anhydride) and is added to a reactor containing hydrogen peroxide (50%, 81.6g, 1.2 moles), acetic anhydride (280 g). The reactor is provided with an acid catalyst selected on the basis of its pKₐ (such as: pKₐ 0 to 5 or using an acid like dichloroacetic acid) in order to promote the Baeyer-Villiger oxidation of cyclododecanone and substantial conversion to laurolactone. This conversion of CDDK to laurolactone is expected to be greater than 99% with selectivity greater than 95% under the reaction conditions herein provided.

Baeyer-Villiger oxidation model reaction:

R-(C=O)-R + H-O-O-H + cat (dichloroacetic acid) = R-(C=O)-O-R

In this case, the R groups of R-(C=O)-R are in a cyclic arrangement and the product, R-(C=O)-O-R, is likewise cyclic and thus a "lactone" or cyclic ester.

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt% to about 5 wt%, but also include individual concentrations (*e.g.*, 1%, 2%, 3%, and 4%) and the sub-ranges (*e.g.*, 0.5%, 1.1%, 2.2%, 3.3%, and 4.4%) within the indicated range. The term "about" can include ±1%, ±2%, ±3%, ±4%, ±5%, ±6%, ±7%, ±8%, ±9%, or ±10%, or more of the numerical value(s) being modified. The value of "about" will not be outside of a reasonable amount considering the teachings of the present disclosure. In addition, the phrase "about 'x' to 'y'" includes "about 'x' to about 'y'".

Many variations and modifications may be made to the above-described embodiments. All such modifications and variations are intended to be included herein within the scope of this disclosure and protected by the following claims.

The invention also encompasses the following embodiments
1. A process for making dodecanedioic acid comprising:
   contacting 1,3-butadiene with a first catalyst and forming cyclododeca-1,5,9-triene;
   oxidizing the cyclododeca-1,5,9-triene using an oxygen containing reagent to form epoxycyclododeca-5,9-diene,
   converting the epoxycyclododeca-5,9-diene into a first mixture comprising cyclododecanol and cyclododecanone; and
   contacting the first mixture with reactants comprising a third catalyst and nitric acid to form a second mixture comprising dodecanedioic acid.
2. The method of embodiment 1, wherein the first catalyst is a homogeneous Ziegler-Natta type catalyst.
3. The method of embodiment 1, wherein the step of oxidizing the cyclododeca-1,5,9-triene is conducted in the presence of a second catalyst.
4. The method of embodiment 1, wherein the second catalyst is selected from:
   hydrocarbon soluble Mo naphthenate, hydrocarbon soluble V naphthenate, hydrocarbon soluble Ti naphthenate, and a combination thereof.
5. The method of embodiment 1, wherein the step of converting the epoxycyclododeca-5,9-diene into a first mixture includes converting the epoxycyclododeca-5,9-diene into the first mixture using a process selected from a selective reduction, rearrangement, or a combination thereof.
6. The method of embodiment 1, wherein the third catalyst is selected from:
   ammonium meta-vanadate and copper nitrate.
7. The method of embodiment 1, wherein cyclododecanol is about 30% to 70% of the first mixture, and wherein the cyclododecanone is about 70% to 30% of the first mixture.
8. The method of embodiment 1, wherein the dodecanedioic acid is about 40% to 90% of the second mixture.
9. The method of embodiment 1, wherein the oxygen containing reagent is a mixture that includes oxygen.
10. The method of embodiment 1, wherein the oxygen containing reagent is a mixture selected from the group consisting of: air enriched with pure oxygen, pure oxygen and pure nitrogen, and a combination thereof.
11. The method of embodiment 10, wherein the mixture includes an amount of oxygen to provide oxygen to the reaction in an amount of about 1% to 99%.
12. The method of embodiment 1, wherein the step of oxidizing the cyclododeca-1,5,9-triene using an oxygen containing reagent further comprises an initiator selected from the group consisting of: AIBN, organic peroxides and, an azoinitiator.
13. The method of embodiment 1, wherein the step of oxidizing the cyclododeca-1,5,9-triene using an oxygen containing reagent is conducted at a reaction temperature of about 50°C to 220°C and at a pressure of about 50 psig to 500 psig.
14. The method of embodiment 1, wherein the step of oxidizing the cyclododeca-1,5,9-triene using an oxygen containing reagent further comprising a metal catalyst.
15. The method of embodiment claim 1, wherein the step of oxidizing the cyclododeca-1,5,9-triene using an oxygen containing reagent is carried out continuously using a fixed bed catalyst.
16. A process for making laurolactone comprising:
   providing a reaction vessel including a solution of cyclododecanone (CDDK) that is dissolved in a about an equal weight of an acid anhydride;
   providing to the vessel an acid catalyst in an amount effective to promote the reaction, wherein the acid catalyst is selected on the basis of its pKₐ such that its pKₐ is about 0 to about 5; and
   introducing hydrogen peroxide to the reaction vessel to promote the Baeyer-Villiger oxidation, wherein about 20% or more of the cyclododecanone (CDDK) is converted to laurolactone.
17. The method of embodiment 16, wherein the acid catalyst is selected from the group consisting of: dichloroacetic acid and a peracid.
18. The method of embodiment 16, wherein the hydrogen peroxide is in an amount of about 1 to 10 moles hydrogen peroxide per mole of CDDK present in the mixture.
19. The method of embodiment 16, wherein amount effective to promote the reaction is in an amount of about 1 to 10 moles acid catalyst per mole of CDDK present in the mixture.
20. A process for making dodecanedioic acid comprising:
   contacting 1,3-butadiene with a first catalyst and forming cyclododeca-1,5,9-triene;
   oxidizing the cyclododeca-1,5,9-triene using an oxygen containing reagent to form epoxycyclododeca-5,9-diene,
   converting the epoxycyclododeca-5,9-diene into a first mixture comprising cyclododecanol and cyclododecanone;
   disposing the cyclododecanone (CDDK) from the first mixture in a reaction vessel, wherein the CDDK is dissolved in a about an equal weight of an acid anhydride;
   providing to the vessel an acid catalyst in an amount effective to promote the reaction, wherein the acid catalyst is selected on the basis of its pKₐ such that its pKₐ is about 0 to about 5; and
   introducing hydrogen peroxide to the reaction vessel to promote the Baeyer-Villiger oxidation, wherein about 20% or more of the (CDDK) is converted to laurolactone.

## Claims

1. A process for making dodecanedioic acid comprising:
contacting 1,3-butadiene with a first catalyst and forming cyclododeca-1,5,9-triene;
oxidizing the cyclododeca-1,5,9-triene using an oxygen containing reagent to form epoxycyclododeca-5,9-diene,
converting the epoxycyclododeca-5,9-diene into a first mixture comprising cyclododecanol and cyclododecanone; and
contacting the first mixture with reactants comprising a third catalyst and nitric acid to form a second mixture comprising dodecanedioic acid; wherein; either
i) the step of oxidizing the cyclododeca-1,5,9-triene is conducted in the presence of a second catalyst and the second catalyst is selected from: hydrocarbon soluble Mo naphthenate, hydrocarbon soluble V naphthenate, hydrocarbon soluble Ti naphthenate, and a combination thereof;
or
ii) the third catalyst is selected from: ammonium meta-vanadate and copper nitrate.

2. The method of claim 1, wherein the first catalyst is a homogeneous Ziegler-Natta type catalyst.

3. The method of claim 1, wherein the step of converting the epoxycyclododeca-5,9-diene into a first mixture includes converting the epoxycyclododeca-5,9-diene into the first mixture using a process selected from a selective reduction, rearrangement, or a combination thereof.

4. The method of claim 1, wherein cyclododecanol is about 30% to 70% of the first mixture, and wherein the cyclododecanone is about 70% to 30% of the first mixture.

5. The method of claim 1, wherein the dodecanedioic acid is about 40% to 90% of the second mixture.

6. The method of claim 1, wherein the oxygen containing reagent is a mixture that includes oxygen.

7. The method of claim 1, wherein the oxygen containing reagent is a mixture selected from the group consisting of: air enriched with pure oxygen, pure oxygen and pure nitrogen, and a combination thereof.

8. The method of claim 8, wherein the mixture includes an amount of oxygen to provide oxygen to the reaction in an amount of about 1% to 99%.

9. The method of claim 1, wherein the step of oxidizing the cyclododeca-1,5,9-triene using an oxygen containing reagent further comprises an initiator selected from the group consisting of: AIBN, organic peroxides and, an azoinitiator.

10. The method of claim 1, wherein the step of oxidizing the cyclododeca-1,5,9-triene using an oxygen containing reagent is conducted at a reaction temperature of about 50°C to 220°C and at a pressure of about 50 psig to 500 psig.

11. The method of claim 1, wherein the step of oxidizing the cyclododeca-1,5,9-triene using an oxygen containing reagent further comprising a metal catalyst.

12. The method of claim 1, wherein the step of oxidizing the cyclododeca-1,5,9-triene using an oxygen containing reagent is carried out continuously using a fixed bed catalyst.
